(19) Europäisches Patentamt
European Patent Office
Office européen des brevets

(11) Veröffentlichungsnummer: **0 183 092**
**A1**

(12) # EUROPÄISCHE PATENTANMELDUNG

(21) Anmeldenummer: **85114024.4**

(22) Anmeldetag: **05.11.85**

(51) Int. Cl.[4]: **C 07 D 239/36**

(30) Priorität: **15.11.84 DE 3441789**

(43) Veröffentlichungstag der Anmeldung:
**04.06.86 Patentblatt 86/23**

(84) Benannte Vertragsstaaten:
**CH DE FR GB LI**

(71) Anmelder: **BAYER AG**
**Konzernverwaltung RP Patentabteilung**
**D-5090 Leverkusen 1 Bayerwerk(DE)**

(72) Erfinder: **Schündehütte, Karl Heinz, Prof. Dr.**
**Klief 75**
**D-5090 Leverkusen 3(DE)**

(72) Erfinder: **Herd, Karl Josef, Dr.**
**Am Gartenfeld 66**
**D-5068 Odenthal(DE)**

(54) **5,6-Dichlor-4-hydroxy-2-trichlormethylpyrimidin.**

(57) 5,6-Dichlor-4-hydroxy-2-trichlormethylpyrimidin erhält man durch Umsetzung von 4-Chlor-2-methyl-6-hydroxypyrimidin mit Chlor in inerten Lösungsmitteln bei Temperaturen von etwa 100 - 160°C.

EP 0 183 092 A1

Croydon Printing Company Ltd.

BAYER AKTIENGESELLSCHAFT 5090 Leverkusen, Bayerwerk
Konzernverwaltung RP
Patentabteilung My-klu/c

# 5,6-Dichlor-4-hydroxy-2-trichlormethylpyrimidin

Gegenstand der vorliegenden Erfindung ist das 5,6-Dichlor-4-hydroxy-2-trichlormethylpyrimidin der Formel

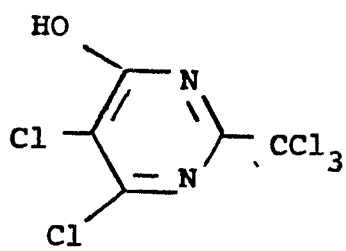

(I)

sowie ein Verfahren zu seiner Herstellung.

Formel (I) gibt uns eine der möglichen tautomeren Formen an.

Das Verfahren besteht darin, daß man das 4-Chlor-2-methyl-6-hydroxypyrimidin der Formel

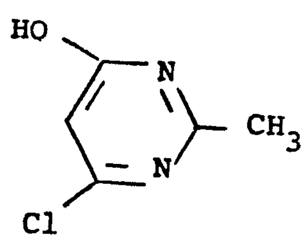

(II)

in einem inerten Lösungsmittel, z.B. Sulfolan oder o-Dichlorbenzol, mit Chlor bei erhöhter Temperatur, beispielsweise 50 - 200°C, vorzugsweise bei 100° bis 160°C, umsetzt.

(I) stellt ein wertvolles Zwischenprodukt zur Herstellung von Reaktivkomponenten für Farbstoffe dar. Durch Chlorierung von (I) mit z.B. Phosphoroxychlorid, Phosphorpentachlorid, Thionylchlorid, Phosgen oder anderen Chlorierungsmitteln sowie Mischungen aus diesen ist beispielsweise in einfacher Weise das 4,5,6-trichlor-2-trichlormethylpyrimidin (III) erhältlich. Durch nachfolgende Umsetzung von (III) mit Fluorwasserstoff oder Kaliumfluorid in an sich bekannter Weise erhält man das 5-Chlor-4,6-difluor-2-trichlormethylpyrimidin bzw. das 5-Chlor-4,6-difluor-2-fluordichlormethylpyrimidin, bekannte Reaktivkomponenten für die Herstellung von Reaktivfarbstoffen.

Herstellungsbeispiele:

5,6-Dichlor-4-hydroxy-2-trichlormethylpyrimidin

144,5 g 4-Chlor-2-methyl-6-hydroxypyrimidin werden in 250 ml o-Dichlorbenzol auf 100°C erwärmt. Bei dieser Temperatur werden zunächst 71.0 g Chlor zudosiert; danach steigert man die Temperatur auf 140°C und leitet weitere 220 g Chlor ein. Die Chlorierungsdauer beträgt ca. 6 Stunden. Es resultiert eine blaßgelbe Lösung, aus der das o-Dichlorbenzol abdestilliert wird. Der Rückstand erstarrt bei ca. 100°C zu einer kristallinen Masse, die sich aufgrund von analytischen und spektroskopischen Daten als 5,6-Dichlor-4-hydroxy-2-trichlormethylpyrimidin erweist. Es werden 282,5 g isoliert. Eine umkristallisierte Probe schmilzt bei 132-134°C (Zers.).

| | | | | | |
|---|---|---|---|---|---|
| Analyse: | Ber.: | C 21.2 | H 0.4 | N 9.9 | Cl 62.8 |
| | Gef.: | C 21.0 | H 0.3 | N 9.8 | Cl 63.0 |

4,5,6-Trichlor-2-trichlormethylpyrimidin

Zur obigen Lösung des 5,6-Dichlor-4-hydroxy-2-trichlormethylpyrimidins in o-Dichlorbenzol (d.h. die Pentachlorverbindung kann ohne Zwischenisolierung weiterverarbeitet werden) gibt man 6 g Formylmethylstearylamin zu und leitet bei 90 - 100° Phosgen ein. Nachdem innerhalb von 6 bis 8 Stunden 110 g Phosgen verbraucht sind, ist die Umsetzung beendet. Im Vakuum wird das o-Dichlorbenzol abdestilliert, und anschließend der Rückstand bei 158°C/20 Torr fraktioniert. Es werden 271 g 4,5,6-Trichlor-2-trichlormethylpyrimidin erhalten.

Patentansprüche:

1. 5,6-Dichlor-4-hydroxy-2-trichlormethylpyrimidin der Formel

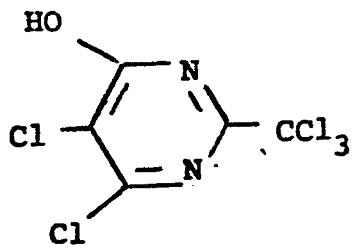

2. Verfahren zur Herstellung von 5,6-Dichlor-4-hydroxy-2-trichlormethylpyrimidin, dadurch gekennzeichnet, daß man 4-Chlor-2-methyl-6-hydroxypyrimidin der Formel

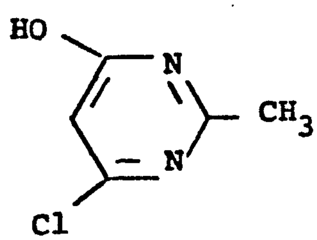

in einem inerten Lösungsmittel mit Chlor bei 50 - 200°C umsetzt.

3. Verfahren zur Herstellung von 4,5,6-Trichlor-2-trichlormethylpyrimidin, dadurch gekenn-

zeichnet, daß man 5,6-Dichlor-4-hydroxy-2-trichlormethylpyrimidin mit Chlorierungsmitteln umsetzt.

4. Verfahren nach Anspruch 3, dadurch gekennzeichnet, daß man als Chlorierungsmittel Phosphoroxychlorid, Phosphorpentachlorid, Phosphortrichlorid, Thionylchlorid, Phosgen oder Mischungen dieser Verbindungen verwendet.

5. Verfahren zur Herstellung von 4,5,6-Trichlor-2-trichlormethylpyrimidin, dadurch gekennzeichnet, daß man 4-Chlor-2-methyl-4-hydroxypyrimidin zunächst mit Chlor in inerten Lösungsmitteln bei 50 - 200°C umsetzt und anschließend ohne Zwischenisolierung mit Phosphorpentachlorid, Phosphoroxychlorid, Phosphortrichlorid, Phosgen, Thionylchlorid oder Mischungen dieser Verbindungen.

0183092

Europäisches Patentamt

# EUROPÄISCHER RECHERCHENBERICHT

Nummer der Anmeldung

EP 85 11 4024

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | DE-A-2 026 819 (FARBENFABRIKEN BAYER AG) * Ansprüche 1, 2 * | 1 | C 07 D 239/36 |
| P,A | EP-A-0 127 827 (BAYER AG) * Beispiele * | 4 | |
| A | CHEMICAL ABSTRACTS, Band 41, Nr. 1, 10. Januar 1947, Spalten 131, 132, Easton, Pa., US; F.R. BASFORD et al.: "Synthetic antimalarials. VIII. Some 4-arylamino-6-aminoalkylamino-2-methylpyrimidines", & JOURNAL OF THE CHEMICAL SOCIETY, 1946, Seiten 713-720, London, GB * Spalte 131 d * | | |
| | | | **RECHERCHIERTE SACHGEBIETE (Int Cl 4)** |
| | | | C 07 D 239/36 |

Der vorliegende Recherchenbericht wurde für alle Patentansprüche erstellt

| Recherchenort | Abschlußdatum der Recherche | Prüfer |
|---|---|---|
| BERLIN | 05-02-1986 | HASS C V F |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82